# EUROPÄISCHE PATENTSCHRIFT

(11) **EP 2 167 863 B1**
(45) Veröffentlichungstag und Bekanntmachung des Hinweises auf die Patenterteilung: **01.01.2014**
(21) Anmeldenummer: 08784960.0
(22) Anmeldetag: 22.07.2008
(51) Int. Cl.: F16M 11/14, A61B 19/00

(54) **VERBINDUNGSVORRICHTUNG**
CONNECTION APPLIANCE
DISPOSITIF DE LIAISON

(30) Priorität: 23.07.2007 DE 102007035922
(43) Veröffentlichungstag der Anmeldung: 31.03.2010
(73) Patentinhaber: CWW-MED AG, 6302 Zug (CH)
(72) Erfinder: BRECHT, Jürgen, 76676 Graben Neudorf (DE); STRAUSS, Christian, 06120 Halle/Saale (DE); ZIAJA, Hermann, 90559 Burgthann (DE)
(74) Vertreter: Witte, Weller & Partner
(86) Internationale Anmeldenummer: PCT/EP2008/005997
(87) Internationale Veröffentlichungsnummer: WO 2009/012978

(56) Entgegenhaltungen:
- DE-A1- 2 048 592
- DE-A1- 19 526 915
- FR-A- 2 711 506
- US-A- 4 807 618

## Beschreibung

Die vorliegende Erfindung betrifft eine Verbindungsvorrichtung zum verstellbaren Verbinden einer chirurgischen Einrichtung, vorzugsweise einer Kopfhalterung, die mit einem ersten Ende der Verbindungsvorrichtung verbindbar ist, mit einem Anbauteil an einer stationären Einrichtung, vorzugsweise einem Operationstisch, das mit einem zweiten Ende der Verbindungsvorrichtung verbindbar ist, mit einem ersten Haltearm, einem zweiten Haltearm, der gelenkig mit dem ersten Haltearm verbunden ist, wobei dem ersten Haltearm eine Kugelgelenkaufnahme und dem zweiten Haltearm ein Kugelgelenkkopf zugeordnet sind, die zusammen ein Kugelgelenk bilden, einer Klemmeinrichtung zur lösbaren Klemmung des Kugelgelenkkopfs in der Kugelgelenkaufnahme, die einen dem Kugelgelenkkopf zugeordneten Klemmschuh aufweist, der über ein Vorspannungselement mit einer Klemmkraft beaufschlagt wird, um die Klemmung zu erzielen, und einer betätigbaren ersten Hydraulikeinrichtung, die der Klemmeinrichtung zugeordnet ist und - bei Betätigung - die Klemmeinrichtung mit einer der Klemmkraft entgegengesetzten Kraft beaufschlagt, wobei die Hydraulikeinrichtung einen Hydraulikzylinder und einen darin verlagerbar angeordneten Hydraulikkolben aufweist, der mit der Klemmeinrichtung verbunden ist.

Verbindungsvorrichtungen der vorgenannten Art sind bekannt. Beispielsweise zeigt die Druckschrift DE 102 34 271 A1 eine solche Vorrichtung. Auch die Druckschriften FR 2 660 714 A1 oder EP 0 868 885 A1 zeigen Verbindungsvorrichtungen, die Kugelgelenke aufweisen. Schließlich ist auf die Druckschrift DE 10 2005 027 882 zu verweisen, die ebenfalls eine Verbindungsvorrichtung offenbart. Auch die Druckschrift DE 198 45 625 C1 zeigt eine Verbindungsvorrichtung.

Derartige Verbindungsvorrichtungen können dazu verwendet werden, eine chirurgische Kopfhalterung an einem Anbauteil, das seinerseits am Operationstisch befestigt ist, zu fixieren. Die Kopfhalterung selbst wird eingesetzt, um den Kopf eines Patienten während eines neurochirurgischen Eingriffs in einer definierten Position relativ zum Operationstisch zu halten.

Die chirurgische Kopfhalterung ist im Allgemeinen um zwei Achsen drehbar an einem Trägerteil befestigt, das seinerseits um eine Drehachse drehbar an der Verbindungsvorrichtung befestigt ist. Das am Operationstisch angebrachte Anbauteil ist ebenfalls um eine Achse drehbar an der Verbindungsvorrichtung gehalten.

In der genannten DE 198 46 625 C1 wird die Klemmung des Anbauteils und des Trägerteils innerhalb der Verbindungsvorrichtung mechanisch über einen manuell betätigbaren Hebel durch Klemmung erreicht.

Mittlerweile sind auch hydraulische Klemmungen bekannt, bspw. aus der zuvor genannten DE 10 2005 027 885 der Anmelderin.

DE 195 26 915 offenbart eine Verbindungsvorrichtung gemäß dem Oberbegriff des Anspruchs 1.

Der Zuverlässigkeit der Klemmung, ob mechanisch oder hydraulisch, kommt sehr große Bedeutung zu, da ein unbeabsichtigtes Lösen der Klemmung fatale Folgen haben kann, die im schlimmsten Fall zum Tode des Patienten führen können. Darüber hinaus muss die Bedienung der Verbindungsvorrichtung einfach sein, um den Operateur zu entlasten.

Vor diesem Hintergrund besteht die Aufgabe der vorliegenden Erfindung darin, die Eingangs genannte Verbindungsvorrichtung so weiterzubilden, dass einerseits eine sehr hohe Klemmkraft und damit eine sichere Verbindung möglich wird und andererseits die Handhabung für den Operateur einfach ausfällt.

Diese Aufgabe wird von der genannten Verbindungsvorrichtung dadurch gelöst, dass eine Klemmkraftübersetzungseinrichtung vorgesehen ist, die zwischen Vorspannungselement und Klemmschuh angeordnet ist und die von dem Vorspannungselement aufgebrachte Kraft in eine größere auf den Klemmschuh wirkende Klemmkraft übersetzt.

Das heißt mit anderen Worten, dass nicht wie beim bisherigen Stand der Technik alleine eine Feder für die erforderliche Klemmkraft sorgt, um die beiden Haltearme in ihrer eingestellten Lage zueinander zu halten, sondern dass diese Klemmkraft von der Klemmkraftübersetzungseinrichtung in eine größere Kraft umgesetzt wird, die dann auf den Klemmschuh und folglich den Kugelgelenkkopf wirkt. Üblicherweise bedeutet eine Übersetzung der Kraft, dass der Federweg gegenüber dem Weg des Klemmschuhs deutlich ansteigt.

Der Vorteil dieser Klemmkraftübersetzungseinrichtung liegt darin, dass eine sehr hohe auf den Kugelgelenkkopf wirkende Klemmkraft erzeugt werden kann, ohne dass die Abmessungen der Feder und damit des gesamten Haltearms zu stark anwachsen würden. Die Verbindungsvorrichtung baut somit weiterhin sehr klein und kompakt.

Zum Lösen dieser festen Klemmung ist die erste Hydraulikeinrichtung vorgesehen, die vom Operateur, bspw. über einen Fußhebel, betätigt wird und eine der Klemmkraft entgegengesetzte Kraft erzeugt.

Dadurch, dass die Klemmkraft nicht alleine von dem Vorspannungselement, üblicherweise einer Feder, aufgebracht werden muss, sind sehr viel höhere Klemmkräfte realisierbar, so dass die Klemmung selbst deutlich sicherer wird. Mit Hilfe der Hydraulikeinrichtung lässt sich die Klemmung sehr einfach und ohne große Kraftanstrengung des Operateurs leicht lösen, so dass eine Neupositionierung der Kopfhalterung einfach und schnell durchführbar ist. Insbesondere ist der Operateur nicht dazu gezwungen, die Klemmkraft über einen Hebel an der Verbindungsvorrichtung manuell aufzubringen, wofür er üblicherweise beide Hände benutzen musste. Stattdessen ermöglicht die erfindungsgemäße Verbindungsvorrichtung, dass der Operateur - wenn überhaupt - nur eine Hand benötigt, um die Hydraulikeinrichtung zu betätigen.

Darüber hinaus ist dafür Sorge getragen, dass auch bei Ausfall der ersten Hydraulikeinrichtung die Klemmung des Kugelgelenkkopfs in der Kugelgelenkaufnahme nicht verloren geht, da die Hydraulikeinrichtung nur zum Lösen vorgesehen ist.

Bei einer bevorzugten Weiterbildung weist die Klemmkraftübersetzungseinrichtung eine zweite Hydraulikeinrichtung mit einem Zylinder und einem darin verlagerbar angeordneten Kolben auf, wobei der Klemmschuh den Kolben bilden, und weist die Klemmeinrichtung ein Betätigungselement auf, das in den Zylinder hineinragt und durch dessen Verlagerung eine Verlagerung des Klemmschuhs herbeiführt.

Das bedeutet mit anderen Worten, dass die Klemmkraftübersetzungseinrichtung auf der Basis eines Hydraulikzylinders arbeitet, wobei die Kopplung zwischen dem Betätigungselement und dem Klemmschuh über eine Hydraulikflüssigkeit erfolgt. Durch die Größenverhältnisse von Betätigungselement und Klemmschuh wird ein langer Weg des Betätigungselements in Längsrichtung mit geringer Kraft in eine Bewegung des Klemmschuhs mit kurzem Weg in Längsrichtung aber mit hoher Kraft umgesetzt. Durch Eintauchen des Betätigungselements in den Zylinderinnenraum wird der Klemmschuh so weit bewegt, dass die Volumenvergrößerung des Zylinderinnenraums dem Volumen des hineinragenden Betätigungselements entspricht.

Bei einer bevorzugten Weiterbildung weist die erste Hydraulikeinrichtung ein Anschlagelement auf, das in den Hydraulikzylinder hineinragt und den Verlagerungsweg des Kolbens begrenzt.

Das heißt mit anderen Worten, dass der Weg zum Lösen des Klemmschuhs durch das Anschlagelement begrenzt wird. Der Vorteil liegt darin, dass durch eine Begrenzung der Weg zum Lösen des Klemmschuhs auf einen minimalen Wert gesetzt werden kann, so dass einerseits nur wenig Hydraulikflüssigkeit in die erste Hydraulikeinrichtung strömen muss und andererseits die Klemmung sehr schnell wieder erreicht wird, wenn der Hydraulikdruck weggenommen wird.

Eine sehr genaue und schnelle Positionierung ist damit erreichbar.

In einer bevorzugten Weiterbildung ist das Anschlagelement von außen einstellbar, wobei das Anschlagelement einen äußeren rohrförmigen Abschnitt mit Innengewinde und einen inneren Abschnitt mit Außengewinde aufweist, die derart zusammenwirken, dass sich bei einer Drehung des inneren Abschnitts der äußere Abschnitt in Längsrichtung verlagert. Es ist weiter bevorzugt, dass ein Schneckgetriebe vorgesehen ist, dessen Schnecke von außen betätigbar ist und mit einem am inneren Abschnitt des Anschlagelements angebrachten Zahnrad zusammenwirkt.

Mit Hilfe dieser Maßnahmen ist eine Einstellung des Anschlagelements in Längsrichtung besonders einfach und genau von außen möglich.

Bei einer bevorzugten Weiterbildung weist jeder Haltearm ein zylindrisches Gehäuse auf, in dem die Klemmeinrichtung und die erste und die zweite Hydraulikeinrichtung untergebracht sind. Vorzugsweise weist die Klemmeinrichtung einen flanschartigen Abschnitt auf, an dem sich ein Ende des Vorspannungselements, insbesondere einer Feder, abstützt, während sich das andere Ende des Vorspannungselements am Gehäuse abstützt.

Diese Maßnahmen haben sich in konstruktiver Sicht als besonders vorteilhaft herausgestellt.

Bei einer bevorzugten Weiterbildung ist die Kugelgelenkaufnahme an einem Ende eines Haltearms anbringbar, insbesondere anschraubbar. Es ist weiter bevorzugt, dass der Kugelgelenkkopf am anderen Ende eines Haltearms anbringbar, insbesondere anschraubbar ist.

Diese Maßnahmen haben den Vorteil, dass sich die Haltearme schnell an unterschiedliche Gegebenheiten anpassen lassen, da bspw. ein Kugelgelenkkopf an einem Ende eines Haltearms schnell durch ein anderes Bauelement ersetzt werden kann, bspw. ein Bauelement zum Befestigen der Kopfhalterung.

Bei einer bevorzugten Weiterbildung sind zumindest drei Haltearme miteinander gelenkig verbunden.

Mit Hilfe von mehreren miteinander gelenkig verbundenen Haltearmen kann der Grad der Einstellbarkeit bspw. einer Kopfhalterung erhöht werden.

Weitere Vorteile und Ausgestaltungen der Erfindung ergeben sich aus der Beschreibung und der beiliegenden Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Hierbei sei insbesondere darauf hingewiesen, dass das zuvor erwähnte Anschlagelement in der ersten Hydraulikeinrichtung auch ohne die erfindungsgemäße Klemmkraftübersetzungseinrichtung einsetzbar ist. Es ist also nicht notwendiger Bestandteil der Klemmkraftübersetzungseinrichtung, sondern kann auch ohne eine solche Klemmkraftübersetzungseinrichtung vorteilhaft in einer Verbindungsvorrichtung eingesetzt werden.

Die Erfindung wird nun anhand eines Ausführungsbeispiels unter Bezugnahme auf die begleitende Zeichnung näher erläutert. Dabei zeigen:
- Fig. 1: eine schematische perspektivische Darstellung einer Verbindungsvorrichtung mit drei Haltearmen, die gelenkig miteinander verbunden sind,
- Fig. 2: eine Querschnittsansicht eines Haltearms der Verbindungsvorrichtung im geklemmten Zustand,
- Fig. 3: eine schematische Schnittdarstellung eines Haltearms in geöffnetem, d.h. nicht geklemmtem Zustand, und
- Fig. 4: eine schematische Schnittdarstellung eines Haltearms in geöffnetem Zustand entsprechend einer alternativen Ausgestaltung

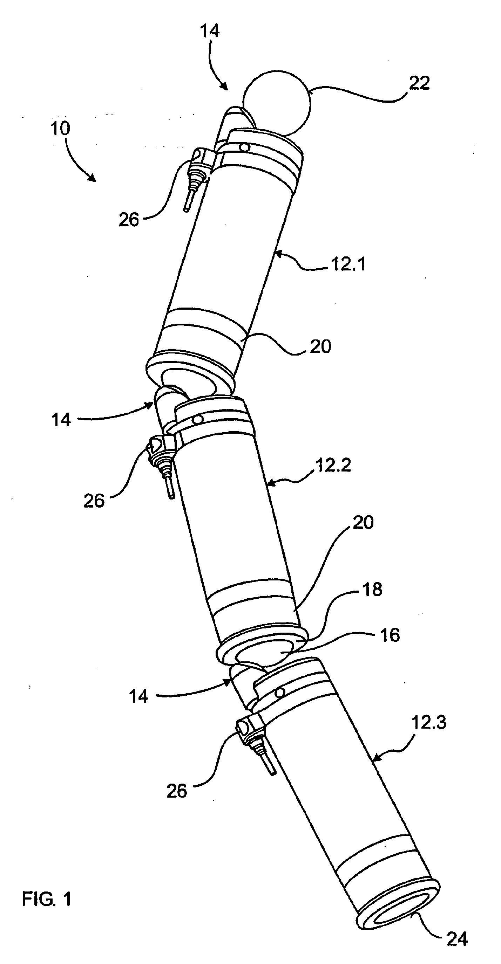

In Fig. 1 ist eine Verbindungsvorrichtung in schematischer Darstellung gezeigt und mit dem Bezugszeichen 10 gekennzeichnet. Die Verbindungsvorrichtung 10 umfasst beispielhaft drei Haltearme 12.1, 12.2 und 12.3, die gelenkig miteinander verbunden sind. Die Anzahl der eingesetzten Haltearme 12 kann auch zwei oder mehr als drei betragen.

Die Verbindungsvorrichtung 10 dient dazu, eine chirurgische Einrichtung, vorzugsweise eine so genannte Kopfhalterung an einem Anbauteil an einer stationären Einrichtung, vorzugsweise einem Operationstisch zu befestigen, so dass die Kopfhalterung durch die gelenkige Verbindung der einzelnen Haltearme 12 justier- und positionierbar ist. Die Kopfhalterung kann bspw. an einem Ende 22 der Verbindungsvorrichtung angebracht werden, während die Verbindungsvorrichtung 10 mit ihrem gegenüberliegenden Ende 24 dann am Operationstisch befestigt ist.

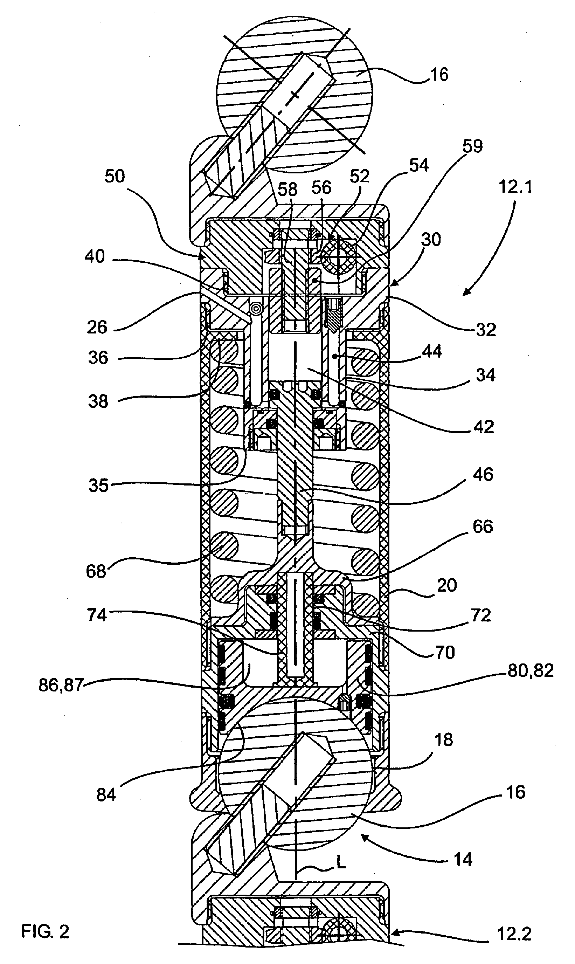

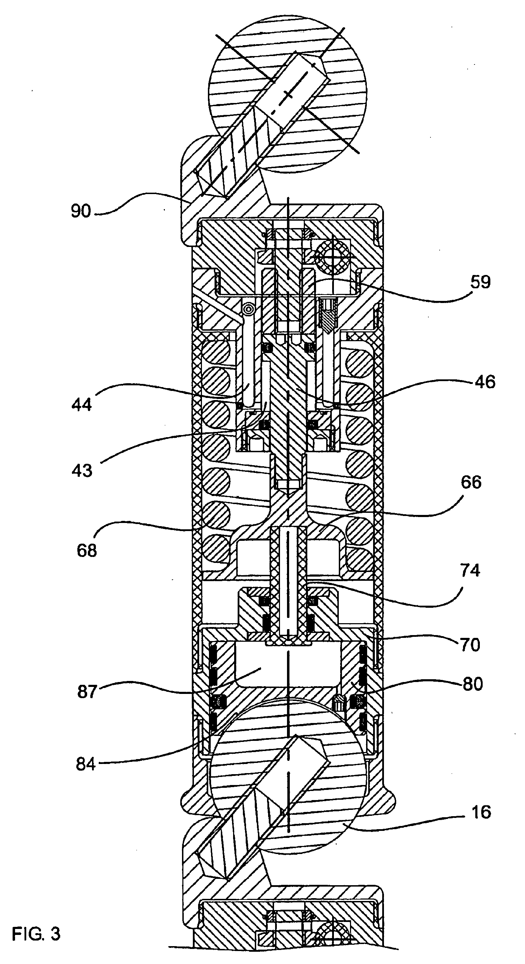

Die gelenkige Verbindung der einzelnen Haltearme 12 miteinander erfolgt über Kugelgelenke 14, die einen Kugelgelenkkopf 16 umfassen, der in einer Kugelgelenkaufnahme 18 des benachbarten Haltearms liegt. Über dieses Kugelgelenk 14 kann der Haltearm 12 sowohl um seine Längsachse, die durch ein zylindrisches Gehäuse 20 definiert wird, gedreht werden als auch in alle Richtungen geschwenkt werden. Im Inneren des zylindrischen Gehäuses 20 eines Haltearms ist eine Vorrichtung vorgesehen, die das Kugelgelenk 14 festklemmt, so dass der entsprechende Haltearm fest justiert und nicht mehr bewegbar ist. Die Beschreibung dieser Vorrichtung erfolgt später mit Bezug auf die Fig. 2 und 3.

Schließlich ist in Fig. 1 noch zu erkennen, dass am zylindrischen Gehäuse 20 jedes Haltearms 12 ein Anschluss 26 vorgesehen ist, der als Hydraulikanschluss dient und das Anbringen eines Hydraulikschlauchs erlaubt. Über diese Hydraulik lässt sich die zuvor erwähnte Klemmung bzw. das Lösen der Klemmung des Kugelgelenks bewirken. Hierzu wird üblicherweise ein Fußschalter vorgesehen, der in den Figuren jedoch nicht gezeigt ist. Der Fußschalter ermöglicht den Benutzern der Verbindungsvorrichtung, den über den Hydraulikanschluss 26 in den entsprechenden Haltearm 12 eingebrachten Hydraulikdruck zu erhöhen, um damit die Klemmung zu lösen. Dies wird zum Justieren und Einstellen der am Ende 22 der Verbindungsvorrichtung angebrachten chirurgischen Einrichtung notwendig. Sobald der Hydraulikdruck weggenommen wird, erfolgt dann wiederum eine Klemmung der Kugelgelenke, so dass die eingestellte Position gehalten werden kann. Üblicherweise sind alle in der Fig. 1 gezeigten drei Hydraulikanschlüsse 26 mit einem Fußschalter verbunden, wobei jedoch andere Lösungen ebenfalls denkbar sind.

Der Aufbau eines Haltearms 12 wird nachfolgend anhand der Fig. 2 und 3 beispielhaft für den Haltearm 12.1 erläutert. Hierbei ist anzumerken, dass der Aufbau der anderen Haltearme 12.2 und 12.3 identisch zu dem Aufbau des Haltearms 12.1 ist.

In den Fig. 2 und 3 ist der Haltearm 12.1 in Schnittdarstellung und zwei unterschiedlichen Zuständen gezeigt, nämlich in einer Klemmposition (Fig. 2) und einer Einstellposition (Fig. 3).

In Fig. 2 ist zu erkennen, dass der Haltearm 12.1 ein zylindrisches Gehäuse 20 aufweist, an dessen - in der Fig. 2 - oberen Ende eine Hydraulikeinheit 30 aufgesetzt ist. Diese Hydraulikeinheit 30 umfasst den bereits zuvor erwähnten Hydraulikanschluss 26 und bildet einen zylindrischen Deckel 32, der das zylindrische Gehäuse 20 nach oben abschließt. Der zylindrische Deckel 32 ist in eine Aufnahme 36 im Gehäuse 20 eingebracht, vorzugsweise eingeschraubt, und liegt auf einem flanschartigen Abschnitt 38 des Gehäuses 20 auf oder ist mit einem kleinen Abstand davon getrennt.

An dem zylindrischen Deckel 32 ist ein rohrförmiger Fortsatz 34 angebracht bzw. integraler Bestandteil, der durch eine Öffnung des flanschartigen Abschnitts 38 des Gehäuses 20 in dessen Innenraum hineinragt.

Dieser rohrförmige Fortsatz 34 ist an seinem unteren Ende mit einem Deckel 35 abgeschlossen und bildet einen Hydraulikzylinder 42. Der rohrförmige Fortsatz 34 besitzt neben dem zylindrischen Innenraum einen Ringraum 44, der in Fluidverbindung mit dem Hydraulikanschluss 26 sowie dem Hydraulik-Innenraum 43 (vgl. Fig. 3) steht.

Der obere Teil des Hydraulikzylinders 42 ist durch eine Anschlageinheit 50 abgeschlossen, die in eine topfförmige Aufnahme 40 des zylindrischen Deckels 32 eingebracht, vorzugsweise eingeschraubt ist.

Die Anschlageinheit 50 umfasst ein Schneckengetriebe 52, das eine Schnecke 54 und ein mit dieser kämmendes Zahnrad 56 umfasst. Das Zahnrad 56 ist mit einer Schraube 58 verbunden, die sich in Längsrichtung L erstreckt. Mit dieser Schraube 58 wirkt eine Anschlaghülse 59 zusammen, die in den Hydraulikzylinder 42 hineinragt. Durch Drehen der Schnecke 54, die von außen betätigbar ist, kann diese Anschlaghülse 59 in Längsrichtung verlagert werden.

Die Anschlaghülse 59 dient als Anschlag für einen Hydraulikkolben 46, der teilweise im Inneren des Hydraulikzylinders 42 liegt. Der Hydraulikkolben 46 ist in Längsrichtung L verlagerbar, wobei eine Bewegung nach oben durch Einbringen von Hydraulikflüssigkeit in den Ringraum 44 und den Innenraum 43 erfolgt. Die Anschlaghülse 59 sorgt hierbei dafür, dass der Hydraulikkolben 46 nicht zu weit nach oben bewegt wird. Sie begrenzt folglich den Verstellweg des Hydraulikkolbens 46.

Am gegenüberliegenden Ende des Hydraulikkolbens 46 ist ein flanschartiger Abschnitt 66 angebracht, dessen Durchmesser in Draufsicht dem Innendurchmesser des zylindrischen Gehäuses 20 entspricht. Wie sich aus Fig. 2 deutlich ergibt, weist dieser flanschartige Abschnitt 66 im Querschnitt eine Topfform auf, wobei am Boden dieses Topfes ein sich in Längsrichtung L erstreckendes zylindrisches Betätigungselement 74 angebracht ist.

Die Fig. 2 lässt weiter erkennen, dass das zylindrische Gehäuse 20 eine Feder 68, in diesem Fall eine Schraubenfeder, aufnimmt, die sich einerseits am flanschartigen Abschnitt 38 des Gehäuses 20 und andererseits an dem flanschartigen Abschnitt 66 abstützt. Die Feder 68 sorgt dafür, dass der Hydraulikkolben 46 nach unten vorgespannt wird, wobei der flanschartige Abschnitt 66 an einem unteren Boden 70, der in das zylindrische Gehäuse 20 eingebracht ist, anschlägt. Dieser Boden 70 besitzt eine zur Längsachse L koaxiale Bohrung 72, durch die das Betätigungselement 74 hindurchgreift. Zusätzlich sind innerhalb dieser Bohrung 72 mehrere Ringdichtungen vorgesehen.

Der Boden 70 weist an seinem unteren Ende eine topfförmige Aufnahme 86 auf, die einen Hydraulik-Innenraum 87 bildet. Dieser Hydraulik-Innenraum wird nach unten von einem Klemmschuh 80 dicht abgeschlossen, der innerhalb der Aufnahme 86 in Längsrichtung verlagerbar ist und somit einen Hydraulikkolben 82 bildet. Der Klemmschuh 80 weist an seiner Unterseite eine kalottenförmige Klemmfläche 84 auf, die mit dem Kugelgelenkkopf 16 zusammenwirkt. Der Kugelgelenkkopf 16, der an dem benachbarten Haltearm 12.2 befestigt ist, liegt in einer Kugelgelenkaufnahme 18 des Haltearms 12.2, die mit dem Boden 70 bspw. verschraubt ist.

Mit Hilfe des Klemmschuhs 80 und seiner Klemmfläche 84 ist es nun möglich, den Kugelgelenkkopf 16 so fest in die Kugelgelenkaufnahme 18 zu drücken, dass eine Bewegung der beiden Haltearme 12.1 und 12.2 zueinander nicht mehr möglich ist.

Die zur Klemmung erforderliche Kraft wird von der Feder 68 aufgebracht, die das Betätigungselement 74 in den Hydraulik-Innenraum 87 bewegt. Da dieser Innenraum mit einem nicht-komprimierbaren Medium gefüllt ist und zudem hermetisch abgedichtet ist, führt die Bewegung des Betätigungselements 74 zu einer Bewegung des Klemmschuhs in gleicher Richtung. Allerdings wird aufgrund der kleineren Grundfläche des Betätigungselements 74 im Vergleich zu der Grundfläche des Klemmschuhs 80 eine Kraftübersetzung erreicht. Die von dem Klemmschuh 80 auf den Kugelgelenkkopf 16 aufgebrachte Klemmkraft ist höher als die Federkraft der Feder 68. Im Gegenzug ist jedoch die Verlagerungsstrecke des Betätigungselements 74 entsprechend größer als die Verlagerungsstrecke des Klemmschuhs 80.

Zum Lösen der Klemmung, d.h. zum Aufwärtsbewegen des Klemmschuhs 80, wird über den Hydraulikanschluss 26 Hydraulikflüssigkeit in den Ringraum 44 und dann in den Innenraum 43 eingebracht. Dadurch wird der Hydraulikkolben 46 gegen die Federkraft der Feder 68 nach oben bewegt und zieht das Betätigungselement 74 aus dem Hydraulik-Innenraum 87 heraus. Dies führt wiederum dazu, dass der Klemmschuh 80 nach oben bewegt wird, allerdings nicht - wie zuvor bereits ausgeführt - um den gleichen Betrag wie das Betätigungselement 74.

Der Weg des Hydraulikkolbens 46 nach oben wird schließlich durch die Anschlaghülse 59 begrenzt, so dass mit Hilfe dieser einstellbaren Anschlaghülse 59 der Verlagerungsweg des Klemmschuhs 80 und damit der Abstand zwischen Klemmfläche 84 und Kugelgelenkkopf 16 auf einen minimalen Wert eingestellt werden kann. Dies hat den Vorteil, dass die Bewegung zwischen Haltearm 12.1 und Haltearm 12.2 zwar ohne Weiteres möglich ist, die Klemmung selbst aber sehr schnell wieder erzielbar ist, da der Verlagerungsweg des Klemmschuhs 80 minimal ist.

In Fig. 3 ist diese Einstellposition des Klemmschuhs 80 deutlich zu erkennen. In dieser Position ist das Betätigungselement 74 nahezu vollständig aus dem Hydraulik-Innenraum 87 herausgezogen. In dieser Position stößt der Hydraulikkolben 46 an der Anschlaghülse 59 an. Durch das nach oben Bewegen des flanschartigen Abschnitts 66 wird die Feder 68 zusammengedrückt, was dazu führt, dass bei einer Wegnahme des Hydraulikdrucks die Feder den flanschartigen Abschnitt 66 wieder nach unten drückt.

In den Figuren ist ferner zu erkennen, dass der Kugelgelenkkopf 16 über eine Schraube an einem Bauteil 90 befestigt ist, das auf die Anschlageinheit 50 aufgesetzt, vorzugsweise aufgeschraubt ist. Die Längsachse der verwendeten Schraube zum Befestigen an dem Bauteil 90 verläuft hierbei schräg zu der Längsachse L.

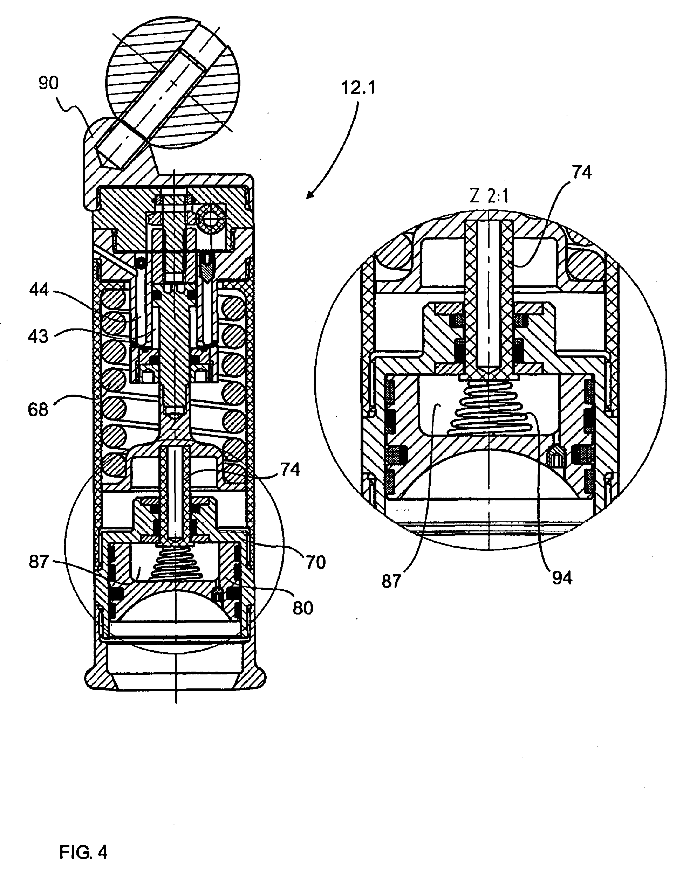

In Fig. 4 ist eine alternative Ausgestaltung des in Fig. 3 gezeigten und beschriebenen Haltearms 12.1 dargestellt. Der Unterschied besteht darin, dass im Hydraulik-Innenraum 87 eine Feder 94, vorzugsweise eine Spiralfeder, vorgesehen ist. Diese Feder 94 stützt sich einerseits am Betätigungselement 74 und andererseits am Klemmschuh 80 ab. Die Feder 94 dient dazu, das Betätigungselement 74 in die Ausgangslage, wie sie in Fig. 4 dargestellt ist, zurückzudrücken, wenn die Feder 68 gespannt wird.

Aus dem Vorgenannten zeigt sich, dass der zuvor beschriebene innere Aufbau eines Haltearms 12 einer Verbindungsvorrichtung 10 entspricht, die einerseits leicht verstellbar aber andererseits die nötige Klemmkraft bereitstellt, um eine feste Klemmung zwischen benachbarten Haltearmen zu erzielen. Dank der Klemmkraftübersetzungseinrichtung, die über die von Betätigungselement 74, Hydraulik-Innenraum 87 und Klemmschuh 80 gebildeten Hydraulikeinrichtungen realisiert wird, kann auch bei einer klein bauenden Feder 68 eine sehr hohe Klemmkraft erzielt werden.

Es versteht sich, dass der in den Fig. 2 und 3 gezeigte Aufbau rein beispielhafter Natur ist und Modifikationen oder Abwandlungen durchaus möglich sind, ohne den durch die Ansprüche abgesteckten Rahmen zu verlassen.

## Patentansprüche

1. Chirurgische Kopfhaltertings-Verbindungsvorrichtung zum verstellbaren Verbinden einer chirurgischen Kopfhalterung mit einem Anbauteil einer stationären Einrichtung, vorzugsweise einem Operationstisch, mit einem ersten Ende und einem zweiten Ende, wobei das erste Ende ausgelegt ist, mit der Kopfhalterung verbindbar zu sein, und das zweite Ende ausgelegt ist, mit dem Anbauteil Verbindbar zu sein, mit
- einem ersten Haltearm (12.1),
- einem zweiten Haltearm (12.2), der gelenkig mit dem ersten Haltearm verbunden ist, wobei dem ersten Haltearm eine Kugelgelenkaufnahme (18) und dem zweiten Haltearm ein Kugelgelenkkopf (16) zugeordnet sind, die zusammen ein Kugelgelenk (14) bilden,
- einer Klemmeinrichtung zur lösbaren Klemmung des Kugelgelenkkopfs (16) in der Kugelgelenkaufnahme (18), die einen dem Kugelgelenkkopf zugeordneten Klemmschuh (80) aufweist, der über ein Vorspannungselement (68) mit einer Klemmkraft beaufschlagt wird, um die Klemmung zu erzielen, und
- einer betätigbaren ersten Hydraulikeinrichtung (30), die der Klemmeinrichtung zugeordnet ist und - bei Betätigung - die Klemmeinrichtung mit einer der Klemmkraft entgegengesetzten Kraft beaufschlagt, wobei die Hydraulikeinrichtung einen Hydraulikzylinder (42) und einen darin verlagerbar angeordneten Hydraulikkolben (46) aufweist, der mit der Klemmeinrichtung verbunden ist,
**gekennzeichnet durch**
eine Klemmkraftübersetzungseinrichtung (74, 82, 86), die zwischen Vorspannungselement (68) und Klemmschuh (80) angeordnet ist und die von dem Vorspannungselement aufgebrachte Kraft in eine größere auf den Klemmschuh (80) wirkende Klemmkraft übersetzt.

2. Chirurgische Kopfhalterungs-Verbindungsvorrichtung nach Anspruch 1, **dadurch gekennzeichnet, dass** die Klemmkraftübersetzungseinrichtung eine zweite Hydraulikeinrichtung mit einem Zylinder und einem darin verlagerbar angeordneten Kolben aufweist, wobei der Klemmschuh (80) den Kolben bildet, und
die Klemmeinrichtung ein Betätigungselement (74) aufweist, dass in den Zylinder hineinragt und durch dessen Verlagerung eine Verlagerung des Klemmschuhs herbeiführt.

3. Chirurgische Kopfhalterungs-Verbindungsvorrichtung nach Anspruch 2, **dadurch gekennzeichnet, dass** die erste Hydraulikeinrichtung (30) ein Anschlagelement (50) aufweist, das in den Hydraulikzylinder hineinragt und den Verlagerungsweg des Kolbens begrenzt.

4. Chirurgische Kopfhalterungs-Verbindungsvorrichtung nach Anspruch 3, **dadurch gekennzeichnet, dass** das Anschlagelement (50) von außen einstellbar ist, wobei das Anschlagelement einen äußeren rohrförmigen Abschnitt mit Innengewinde und einen inneren Abschnitt mit Außengewinde aufweist, die derart zusammenwirken, dass sich bei einer Drehung des inneren Abschnitts der äußere Abschnitt in Längsrichtung verlagert.

5. Chirurgische Kopfbalterungs-Verbindungsvorrichtung nach Anspruch 4, **dadurch gekennzeichnet, dass** ein Schneckengetriebe (52) vorgesehen ist, dessen Schnecke von außen betätigbar ist und mit einem am inneren Abschnitt des Anschlagelements angebrachten Zahnrad zusammenwirkt.

6. Chirurgische Kopfhalterungs-Verbindungsvorrichtung nach einem der vorhergehenden Ansprüche, **dadurch gekennzeichnet, dass** das Vorspannungselement eine Feder, insbesondere eine Schraubenfeder ist.

7. Chirurgische Kopfhalterungs-Verbindungsvorrichtung nach einem der vorhergehenden Ansprüche, **dadurch gekennzeichnet, dass** jeder Haltearm (12) ein zylindrisches Gehäuse aufweist, in dem die Klemmeinrichtung und die erste und die zweite Hydraulikeinrichtung untergebracht sind.

8. Chirurgische Kopfhalterungs-Verbindungsvorrichtung nach Anspruch 6 und 7, **dadurch gekennzeichnet, dass** die Klemmeinrichtung einen flanschartigen Abschnitt aufweist, an dem sich ein Ende der Feder (68) abstützt, während sich das andere Ende der Feder am Gehäuse abstützt.

9. Chirurgische Kopfhalterungs-Verbindungsvorrichtung nach einem der vorhergehenden Ansprüche, **dadurch gekennzeichnet, dass** die Kugelgelenkaufnahme (18) an einem Ende eines Haltearms anbringbar, insbesondere anschraubbar ist.

10. Chirurgische Kopfhalterungs-Verbindungsvorrichtung nach Anspruch 9, **dadurch gekennzeichnet, dass** der Kugelgelenkkopf (16) am anderen Ende eines Haltearms anbringbar, insbesondere anschraubbar ist.

11. Chirurgische Kopfhalterungs-Verbindungsvorrichtung nach einem der vorhergehenden Ansprüche, **dadurch gekennzeichnet, dass** zumindest drei Haltearme miteinander gelenkig verbunden sind.

12. Chirurgische Kopfhalterungs-Verbindungsvorrichtung nach einem der vorhergehenden Ansprüche, **dadurch gekennzeichnet, dass** die erste Hydraulikeinrichtung an eine Druckeinrichtung anschließbar ist, die per Fußbedienung betätigbar ist und den Hydraulikdruck erzeugt.

13. Chirurgische Kopfhalterungs-Verbindungsvorrichtung nach Anspruch 2, **dadurch gekennzeichnet, dass** zwischen Betätigungselement (74) und Klemmschuh (80) eine Feder (94) vorgesehen ist.

## Claims

1. A surgical head support connection device for adjustably connecting a surgical head support to an attachment part of a stationary device, preferably an operating table, comprising a first end and a second end, the first end being adapted to be connectable to the head support and the second end being adapted to be connectable to the attachment part, the surgical head support connection device having
- a first holding arm (12.1),
- a second holding arm (12.2), connected to the first holding arm in a hinged fashion, with a ball and socket joint receptacle (18) being assigned to the first holding arm and a ball and socket joint ball (16) being assigned to the second holding arm, which together form a ball and socket joint (14),
- a clamping apparatus for the detachable clamping of the ball and socket joint ball (16) in the ball and socket joint receptacle (18), the latter having a clamp-type socket (80) which is assigned to the ball and socket joint ball and acted on by a clamping force via a pretensioning element (68) in order to attain the clamping, and
- a first hydraulic apparatus (30) which can be actuated, is assigned to the clamping apparatus and, when operated, loads the clamping apparatus with a force opposing the clamping force, with the hydraulic apparatus having a hydraulic cylinder (42) and a hydraulic piston (46) which is arranged such that it can be displaced therein and connected to the clamping apparatus,
**characterized by**
a clamping force transformation apparatus (74, 82, 86) which is arranged between the pretensioning element (68) and clamp-type socket (80) and transforms the force applied by the pretensioning element into a larger clamping force acting on the clamp-type socket (80).

2. The surgical head support connection device as claimed in claim 1, **characterized in that** the clamping force transformation apparatus has a second hydraulic apparatus with a cylinder and a piston which is arranged such that it can be displaced therein, with the clamp-type socket (80) forming the piston, and
the clamping apparatus has an actuation element (74) which projects into the cylinder and the displacement of which causes a displacement of the clamp-type socket.

3. The surgical head support connection device as claimed in claim 2, **characterized in that** the first hydraulic apparatus (30) has a stop element (50) which projects into the hydraulic cylinder and limits the displacement path of the piston.

4. The surgical head support connection device as claimed in claim 3, **characterized in that** the stop element (50) can be adjusted from the outside, the stop element having an outer tube-shaped section with a female thread and an inner section with a male thread which interact such that a rotation of the inner section displaces the outer section in the longitudinal direction.

5. The surgical head support connection device as claimed in claim 4, **characterized in that** provision is made for a worm drive (52), the worm of which can be actuated from the outside and interacts with a gearwheel attached to the inner section of the stop element.

6. The surgical head support connection device as claimed in one of the preceding claims, **characterized in that** the pretensioning element is a spring, in particular a helical spring.

7. The surgical head support connection device as claimed in one of the preceding claims, **characterized in that** each holding arm (12) has a cylindrical housing in which the clamping apparatus and the first and the second hydraulic apparatus are housed.

8. The surgical head support connection device as claimed in claim 6 and 7, **characterized in that** the clamping apparatus has a flange-like section on which one end of the spring (68) is supported, while the other end of the spring is supported on the housing.

9. The surgical head support connection device as claimed in one of the preceding claims, **characterized in that** the ball and socket joint receptacle (18) can be attached, in particular screwed, to one end of a holding arm.

10. The surgical head support connection device as claimed in claim 9, **characterized in that** the ball and socket joint ball (16) can be attached, in particular screwed, to the other end of a holding arm.

11. The surgical head support connection device as claimed in one of the preceding claims, **characterized in that** at least three holding arms are interconnected in a hinged fashion.

12. The surgical head support connection device as claimed in one of the preceding claims, **characterized in that** the first hydraulic apparatus can be connected to a pressure apparatus which can be operated by foot operation and generates the hydraulic pressure.

13. The surgical head support connection device as claimed in claim 2, **characterized in that** provision is made for a spring (94) between the actuation element (74) and the clamp-type socket (80).

## Revendications

1. Dispositif de liaison chirurgical pour le maintien de la tête, pour relier de manière réglable un système chirurgical de maintien de la tête à une pièce rapportée d'un dispositif stationnaire, de préférence une table d'opération, comprenant une première extrémité et une deuxième extrémité, la première extrémité étant conçue de manière à pouvoir être reliée au système de maintien de la tête, et la deuxième extrémité étant conçue de manière à pouvoir être reliée à la pièce rapportée, comprenant :
- un premier bras de retenue (12.1),
- un deuxième bras de retenue (12.2), qui est relié de manière articulée au premier bras de retenue, un logement d'articulation à rotule (18) étant associé au premier bras de retenue et une tête d'articulation à rotule (16) étant associée au deuxième bras de retenue, lesquels forment ensemble une articulation à rotule (14),
- un dispositif de serrage pour le serrage desserrable de la tête d'articulation à rotule (16) dans le logement d'articulation à rotule (18), qui présente un sabot de serrage (80) associé à la tête d'articulation à rotule, lequel sabot est sollicité par une force de serrage par le biais d'un élément de précontrainte (68), afin de produire le serrage, et
- un premier dispositif hydraulique commandable (30) qui est associé au dispositif de serrage et qui - lors de son actionnement - sollicite le dispositif de serrage avec une force opposée à la force de serrage, le dispositif hydraulique présentant un cylindre hydraulique (42) et un piston hydraulique (46) disposé de manière déplaçable dans celui-ci, lequel est connecté au dispositif de serrage,
**caractérisé par**
un dispositif de transformation de force de serrage (74, 82, 86) qui est disposé entre l'élément de précontrainte (68) et le sabot de serrage (80) et qui transforme la force appliquée par l'élément de précontrainte en une force de serrage plus importante agissant sur le sabot de serrage (80).

2. Dispositif de liaison chirurgical pour le maintien de la tête selon la revendication 1, **caractérisé en ce que** le dispositif de transformation de force de serrage présente un deuxième dispositif hydraulique avec un cylindre et un piston disposé de manière déplaçable dans celui-ci, le sabot de serrage (80) formant le piston, et
le dispositif de serrage présentant un élément d'actionnement (74) qui pénètre dans le cylindre et dont le déplacement provoque le déplacement du sabot de serrage.

3. Dispositif de liaison chirurgical pour le maintien de la tête selon la revendication 2, **caractérisé en ce que** le premier dispositif hydraulique (30) présente un élément de butée (50) qui pénètre dans le cylindre hydraulique et qui limite la course de déplacement du piston.

4. Dispositif de liaison chirurgical pour le maintien de la tête selon la revendication 3, **caractérisé en ce que** l'élément de butée (50) peut être ajusté de l'extérieur, l'élément de butée présentant une portion extérieure tubulaire ayant un filetage interne et une portion intérieure ayant un filetage externe, lesquelles coopèrent l'une avec l'autre de telle sorte que dans le cas d'une rotation de la portion interne, la portion externe se déplace dans la direction longitudinale.

5. Dispositif de liaison chirurgical pour le maintien de la tête selon la revendication 4, **caractérisé en ce qu'**une transmission à vis sans fin (52) est prévue, dont la vis sans fin peut être actionnée de l'extérieur et coopère avec une roue dentée montée sur la portion intérieure de l'élément de butée.

6. Dispositif de liaison chirurgical pour le maintien de la tête selon l'une quelconque des revendications précédentes, **caractérisé en ce que** l'élément de précontrainte est un ressort, notamment un ressort à boudin.

7. Dispositif de liaison chirurgical pour le maintien de la tête selon l'une quelconque des revendications précédentes, **caractérisé en ce que** chaque bras de retenue (12) présente un boîtier cylindrique dans lequel sont montés le dispositif de serrage et le premier et le deuxième dispositif hydraulique.

8. Dispositif de liaison chirurgical pour le maintien de la tête selon les revendications 6 et 7, **caractérisé en ce que** le dispositif de serrage présente une portion en forme de bride, sur laquelle s'appuie une extrémité du ressort (68) tandis que l'autre extrémité du ressort s'appuie sur le boîtier.

9. Dispositif de liaison chirurgical pour le maintien de la tête selon l'une quelconque des revendications précédentes, **caractérisé en ce que** le logement d'articulation à rotule (18) peut être monté, notamment vissé, à une extrémité d'un bras de retenue.

10. Dispositif de liaison chirurgical pour le maintien de la tête selon la revendication 9, **caractérisé en ce que** la tête d'articulation à rotule (16) peut être montée, notamment vissée, à l'autre extrémité du bras de retenue.

11. Dispositif de liaison chirurgical pour le maintien de la tête selon l'une quelconque des revendications précédentes, **caractérisé en ce qu'**au moins trois bras de retenue sont reliés les uns aux autres de manière articulée.

12. Dispositif de liaison chirurgical pour le maintien de la tête selon l'une quelconque des revendications précédentes, **caractérisé en ce que** le premier dispositif hydraulique peut être raccordé à un dispositif de pression, qui peut être actionné par une commande du pied, et qui génère la pression hydraulique.

13. Dispositif de liaison chirurgical pour le maintien de la tête selon la revendication 2, **caractérisé en ce qu'**entre l'élément d'actionnement (74) et le sabot de serrage (80) est prévu un ressort (94).
